# EUROPEAN PATENT APPLICATION

(11) **EP 4 606 885 A1**
(43) Date of publication of application: **27.08.2025**
(21) Application number: 22962871.4
(22) Date of filing: 14.12.2022
(51) Int. Cl.: C12N 1/20, C08J 11/10, B09B 3/60, C12R 1/385

(54) **NOVEL MICROBE HAVING PLASTIC DECOMPOSITION ACTIVITY, AND USE THEREOF**

(30) Priority: 17.10.2022 KR 20220133092
(71) Applicant: Repla Inc., Gyeongsan-si, Gyeongsangbuk-do 38541 (KR)
(72) Inventor: CHOI, Donggeon, Suwon-si Gyeonggi-do 16694 (KR); KIM, Hongrae, Suwon-si Gyeonggi-do 16679 (KR); SUH, Dong Eun, Suwon-si Gyeonggi-do 16698 (KR)
(74) Representative: Schiweck Weinzierl Koch Patentanwälte Partnerschaft mbB
(86) International application number: PCT/KR2022/020317
(87) International publication number: WO 2024/085310

(57) **Abstract**

The present invention relates to a novel microbe that can decompose a plastic and a method of decomposing a plastic using the same. The microbe may decompose at least one plastic selected from the group consisting of PET, PVC, PS, PP, and PE under appropriate incubation conditions and convert the plastic into a low molecular weight substance.

## Description

### [Technical Field]

The present invention relates to a novel microorganism that can degrade various types of plastics and a method of degrading a plastic using the same.

### [Background Art]

Plastics have been rapidly increasing in use based on their light properties, high physical and chemical durability, high processability, and very low price, and the global annual plastic production, which reached 2 million tons in 1950, was approaching 370 million tons as of 2019. In addition, the recent spread and persistence of COVID-19 has led to a surge in the use of personal protective equipment such as disposable masks and gloves, which are mainly made of plastics, an increase in the use of disposable tableware due to the rise in take-out and home delivery food, and an increase in the use of plastic packaging containers due to the preference for online shopping, and therefore the use of plastics is expected to continue to increase in the future (Trends in biodegradation of plastics using microorganisms, BRIC View 2021-T34).

The surge in plastic usage and the depletion of petroleum, which is the raw material for plastics, have created a new need to recycle plastics that are discarded as waste. Plastics are usually collected and recycled together because different types of plastics are used in one product. For this reason, it is difficult to maintain complete purity of each plastic material as a single material due to the impurities of other plastic materials, and as a result, the quality is reduced, and the economic value is evaluated to be approximately 34% lower than that of new plastic. Particularly, it is difficult to economically separate mixed waste plastics mixed in living waste by material, and therefore there is an urgent need to develop technology to sort mixed waste plastics.

Meanwhile, recently, technology has been developed to treat plastics contained in wastewater or waste using microorganisms. For example, Korean Patent No. 10-0350928 discloses a novel microorganism, *Klebsiella pneumoniae* CJ-PVA a (Accession No. KFCC-11126), which grows well under aerobic conditions and has an improved polyvinyl alcohol decomposition ability, and a method of treating polyvinyl alcohol-containing wastewater using the same. In addition, Korean Patent No. 10-0513931 discloses *Microbacterium barkeri* LC (Accession No. KCCM 10507) and a method of biologically decomposing polyvinyl alcohol using the same.

### [Disclosure]

### [Technical Problem]

In the above situation, the inventors of the present invention conducted research to discover plastic-degrading microorganisms, and discovered a novel plastic-degrading microorganism, the *Pseudomonas aeruginosa* repla1 strain, by feeding polystyrene as food to beetle larvae (superworms) and isolating the microorganisms.

Therefore, the present invention aims to provide a novel plastic-degrading microorganism and a method of degrading a plastic using the same.

### [Technical Solution]

The present invention is directed to providing the *Pseudomonas aeruginosa* repla1 strain having plastic degradation activity, which is deposited under Accession No. KACC 81230BP.

In the present invention, the plastic may be one or more type selected from the group consisting of polyethylene terephthalate (PET), polyvinyl chloride (PVC), polystyrene (PS), polypropylene (PP), and polyethylene (PE).

PET is highly transparent and has no taste or smell, making it the most common plastic beverage bottle on the market. PVC is a type of thermoplastic that is strong, rigid or flexible, and wear-resistant. It is used for artificial leather, packaging materials, pipes, electrical insulators, etc., and is plastic that has been used for the longest time under the name "vinyl."

PS is a type of thermoplastic that is lightweight, tasteless, and odorless and is used in household items, toys, electrical insulators, radio and television cases, and packaging materials. PP is polymerized from propylene obtained from petroleum and is widely used in bottles, etc.

PE is a type of thermoplastic that is lightweight and flexible, and is a general-purpose plastic used in everything from industrial materials to everyday items. High-density polyethylene (HDPE) is impact-resistant and cold-resistant, and therefore is mainly used to make shopping bags and pipes. Low-density polyethylene (LDPE) has branches in the polymer, and therefore has a lower density than linear HDPE, and is easy to process due to its good elasticity.

In the present invention, "plastic degradation" means decomposition of a polymeric material constituting a plastic to a low molecular weight intermediate or to an intermediate that can be metabolized by the metabolic pathway of microorganisms.

According to an embodiment of the present invention, the *Pseudomonas aeruginosa* repla1 strain was isolated from the intestine of beetle larvae that were fed with PS (Example 1). However, the *Pseudomonas aeruginosa* repla1 strain is also capable of degrading LDPE and HDPE (Examples 2-1 and 2-4).

The present invention is also directed to providing a method of degrading a plastic, which includes incubating the *Pseudomonas aeruginosa* repla1 strain deposited under Accession No. KACC 81230BP along with a plastic.

The incubation may be performed at 8 to 40 °C for 7 to 60 days after inoculating the *Pseudomonas aeruginosa* repla1 strain into a medium containing a plastic as a carbon source, but the present invention is not limited thereto. Preferably, the incubation is performed at 8 to 37 °C for 7 to 30 days.

In the present invention, the above medium composition, incubation temperature, and incubation time may vary depending on the type of plastic to be degraded, and when the *Pseudomonas aeruginosa* repla1 strain is incubated with a waste plastic or plastic-containing waste under conditions that combine these conditions as process parameters, a specific plastic may be relatively degraded or all plastics may be degraded into reusable small molecule materials.

The plastic added to the medium may be in the form of finely shredded flakes or thin films (waste vinyl) to increase contact with the *Pseudomonas aeruginosa* repla1 strain or a plastic-degrading enzyme secreted by the strain.

According to an embodiment of the present invention, the *Pseudomonas aeruginosa* repla1 strain expresses laccase when using a plastic as a carbon source.

Laccase is a type of multi-copper blue oxidase containing copper and has been found in higher plants, insects, bacteria, and fungi. Laccase catalyzes the oxidation of several substances, particularly phenols and aromatic amines, using the electrons released during the process of reducing oxygen molecules to water. Due to this characteristic, it is being used in various industrial and environmental fields (lignin decomposition, decolorization, compound synthesis, purification of polluted water and oil).

In relation to the degradation of plastics, which are polymeric materials, the catalytic action caused by oxidation may increase the hydrophilicity of the hydrophobic plastic surface, thereby promoting the formation of a microbial biofilm (hydrophilic) and depolymerization (Reference Documents 1 and 2).

The method of degrading a plastic according to the present invention may be applied as a pretreatment process for plastic recycling.

The present invention is also directed to providing a composition for degrading a plastic, which includes the *Pseudomonas aeruginosa* repla1 strain deposited under Accession No. KACC 81230BP, a culture of the strain, or a plastic-degrading enzyme derived from the strain.

As described above, the *Pseudomonas aeruginosa* repla1 strain expresses laccase and uses a plastic as a carbon source, and therefore the strain itself, the culture of the strain, and the strain-derived plastic-degrading enzyme may be used for plastic degradation.

The enzyme having plastic degrading activity may be an extracellular or intracellular material of a microorganism with plastic degrading activity, and may be mass-produced through various genetic recombination.

### [Advantageous Effects]

A microorganism having plastic degrading activity according to the present invention can degrade one or more types of plastics selected from the group consisting of polyethylene terephthalate (PET), polyvinyl chloride (PVC), polystyrene (PS), polypropylene (PP), and polyethylene (PE) under appropriate incubation conditions and convert them into small molecule materials. Therefore, the microorganism having plastic degrading activity according to the present invention can be used in a pretreatment process for plastic recycling.

### [Description of Drawings]

FIG. 1 shows the results of confirming whether or not a plastic-degrading microorganism (hereinafter referred to as repla1) metabolizes polyethylene (PE) after repla1 is incubated according to an example of the present invention in a PE powder-added minimal medium using DCPIP absorbance: Error bars represent the standard deviation of the mean, and an asterisk indicates *p* <0.05 (Mann-Whitney U test).
FIG. 2A shows the results of confirming whether repla1 metabolizes metabolites when repla1 is incubated in a medium containing various metabolites: At indicates a DCPIP absorbance value of each material over time, ACt indicates a DCPIP absorbance value of a control (without metabolite) over time, and error bars represent the standard deviation of the mean.
FIG. 2B shows the results of confirming whether *Escherichia* sp., which does not possess plastic-degrading activity, metabolizes carbon sources when *Escherichia* sp. is incubated in a medium containing various carbon sources: At indicates a DCPIP absorbance value of each material over time, ACt indicates a DCPIP absorbance value of a control (without metabolite) over time, and error bars represent the standard deviation of the mean.
FIG. 3 shows the results of confirming the number of viable cells when repla1 is incubated in a minimal medium containing a plastic film: Error bars represent the standard deviation of the mean, and an asterisk indicates *p* <0.05 (Mann-Whitney U test).
FIGS. 4A and 4B show the results of confirming the formation of a microbial biofilm on the surface of a plastic film after repla1 is incubated in a minimal medium containing a plastic film.
FIG. 4C shows the results of confirming the degree of corrosion on the surface of a plastic film after repla1 is incubated in a minimal medium containing a plastic film.
FIG. 4D shows the results of confirming the degree of corrosion on the surface of a plastic film after a minimal medium containing a plastic film is incubated without repla1.

### [Modes of the Invention]

Hereinafter, one or more exemplary embodiments will be described in more detail through examples. However, these examples are intended to exemplify one or more exemplary embodiments and the scope of the present invention is not limited to these examples.

### Example 1: Isolation of plastic-degrading microorganisms

### 1. Isolation of plastic-degrading microorganisms

By feeding polyethylene (PE) as a food to beetle larvae (superworms) for two weeks, a selective pressure was applied to hydrocarbon-based plastic monomers, ensuring a dominant gut microbiota. To isolate only pure gut microorganisms, the larvae were sterilized with 70% ethanol and washed with 0.9% saline, and the heads and tails were removed to extract the internal organs. The extracted internal organs were cut into pieces with a sterile knife and then mixed with saline, followed by separation of epithelial cells and a supernatant by centrifugation.

A portion of the separated supernatant was added to a minimal medium, and a polystyrene (PS) film was provided as the sole carbon source and energy source to incubate microorganisms with potential plastic degradability by shaking culture under aerobic conditions (25 °C and 180 rpm) for approximately 60 days. The composition of the minimal medium is as follows: pH 6.51, 0.7 g NH₂PO₄, 0.7 g K₂HPO₄, 0.7 g MgSO₄·7H₂O, 1.0 g NH₄NO₃, 0.005 g NaCl, 0.002 g FeSO₄·7H₂O, 0.002 g ZnSO₄·7H₂O, 0.001 g MnSO₄·H₂O per 1 L distilled water. One strain that was grown using a PS film as a carbon source was isolated through pure separation after culture.

### 2. Microbial identification

To identify the pure single strain obtained from the third subculture, 16S rRNA sequencing was performed, identifying the ensured strain as belonging to the genus *Pseudomonas.* The isolated microorganism was named *Pseudomonas aeruginosa* repla1, and deposited under Accession No. KACC 81230BP in the Korean Agricultural Culture Collection (KACC) at the National Institute of Agricultural Science.

Hereinafter, the isolated strain is described as "repla1."

### Example 2: Verification of plastic degradability

### 2-1. Verification of activity of direct plastic metabolism

After repla1 was cultured in a medium containing a nutrient medium (Luria-Bertani broth, LB) for 24 hours, the cells (10⁹ cells/mL) corresponding to a 600 nm absorbance of 1 were recovered. The recovered repla1 cells were washed twice with 0.9% saline to remove the previous culture medium and suspended in the minimal medium.

2,6-dichlorophenolindophenol (DCPIP) oxidizing agent and 8 g/L of low-density polyethylene (LDPE; Mw: up to 4,000, Sigma-Aldrich) powder were added to the minimal medium. Here, the cells were incubated after the repla1 suspension was inoculated at a ratio of 1/100 (10⁷ cells/mL), and DCPIP absorbance was measured every 24 hours. Every 24 hours, a portion of the culture was collected and then the cells and the suspension were separated by centrifugation, and the absorbance (600 nm) of the supernatant was then measured. The DCPIP color change of an experimental group was compared with the two controls below: 1) without PE + with repla1, 2) with PE + without repla1.

DCPIP is reduced by receiving electrons from energy generated by the metabolic activity of microorganisms, changing its color from blue to colorless. This allows the direct plastic degradation and internal metabolic activity of the isolated repla1 to be indirectly evaluated.

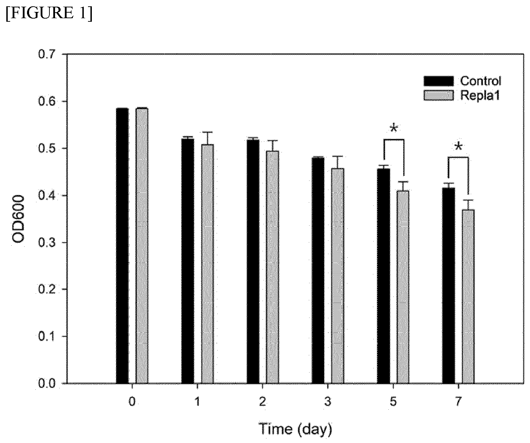

As a result of the absorbance measurement, it can be seen that repla1 can use LDPE powder as the sole carbon and energy source, and generates reduction energy by internal metabolism, and the reaction appears approximately 5 days after the start of culture (FIG. 1). The results are marked with * indicating statistically significant differences compared to the control inoculated with only repla1 alone in the minimal medium (Mann-Whitney U test, *p* <0.05).

### 2-2. Verification of internal metabolism of plastic monomer

In order to metabolize a plastic made in a polymer structure, degradation into monomer units with a low molecular weight (Mw: up to 500) must take place, and the monomers are used in various types of microbial metabolism. Among various monomers, a hydrocarbon structural material ([CnHn]n) is mainly metabolized through a fatty acid degradation pathway (β-oxidation pathway), and the metabolism must occur step by step in the order of alkane, alcohol, aldehyde, and fatty acid (Reference documents 3 to 6).

Here, the ability of repla1 to use plastic monomers was evaluated.

In the same manner as Example 2-1, repla1 was pre-cultured and then suspended in a minimal medium. As the sole energy and carbon source, alkane hydrocarbon, alcohol, aldehyde, fatty acid, or glucose (positive control) was added to the minimal medium. The repla1 suspension was inoculated into the medium at a ratio of 1/100 (10⁷ cells/mL) for incubation, and DCPIP absorbance was measured every 24 hours. Every 24 hours, a portion of the culture medium was collected to separate the cells and the supernatant by centrifugation, and then absorbance (600 nm) of the supernatant was measured. The DCPIP color change of the experimental group was compared with the two controls below: 1) without metabolite + with repla1, 2) with internal metabolite + without repla1.

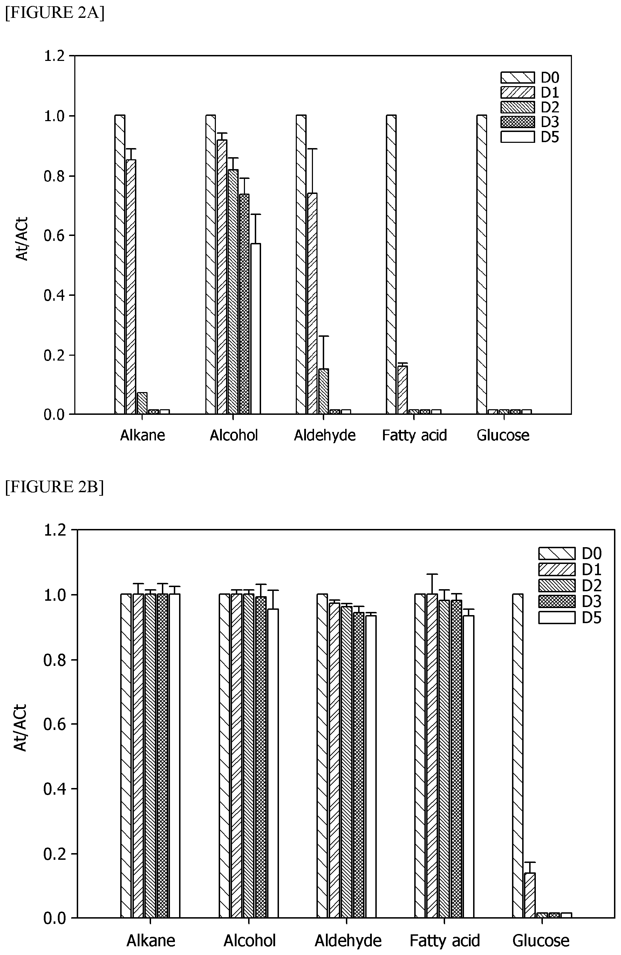

As a result of absorbance measurement, repla1 was able to use all of alkane, alcohol, aldehyde, and fatty acid used in the experiment as carbon sources, which in decreasing order of metabolic rate are a fatty acid, an aldehyde, an alkane, and an alcohol (FIG. 2A). It was confirmed that *Escherichia* sp. with no plastic degradation ability, which was used as a negative control, does not metabolize materials other than glucose (FIG. 2B).

### 2-3. Verification of expression of plastic-degrading enzyme (exozyme)

There are two main methods for degrading polymer-type plastics into monomeric units: a physicochemical method and a biological method. The biological method is an enzyme-associated degradation method, and the enzymes involved in plastic degradation may include: an enzyme that oxidizes a plastic surface and a hydrolytic enzyme that breaks a polymer chain. Representative enzymes associated with surface oxidation include laccase (oxidase), and hydrolytic enzymes include lipase and esterase.

Whether repla1 exhibits the above enzyme activity was confirmed in both a general environment (e.g., nutrient medium) and a special environment (e.g., plastic medium). After culturing repla1 in a minimal medium containing a nutrient medium or plastic film (LDPE and HDPE), the culture was centrifuged and the supernatant was separated to prepare an enzyme sample.

Laccase activity was measured based on the amount of an enzyme required for oxidizing a 1 µM 2,6-dimethoxy phenol (DMP) substrate for 1 minute. 600 µL sodium citrate (100 mM sodium citrate, pH 4.0), 400 µL DMP (10 mM), and a 500 µL enzyme sample were added and mixed well. The enzyme activity per minute was calculated by measuring the change in absorbance (468 nm) for 2 minutes.

The activity of lipase and esterase was assessed by measuring the amount of a product produced by degrading a substrate for 10 minutes. The enzyme sample was reacted with each of 4-nitrophenyl palmitate and 4-nitrophenyl butyrate substrates at 28 °C for 10 minutes, and then the absorbance (410 nm) of the reaction solution was measured. The confirmed enzyme activity of repla1 is shown in Table 1.

**[Table 1]**

| Conditions of incubation | Laccase | Lipase | Esterase |
|---|---|---|---|
| Nutrient medium (LB) | X | O | O |
| Minimal medium + plastic film | O | X | X |

The experimental result showed that repla1 uses laccase when using a plastic as a carbon source.

### 2-4. Observation of growth of strain using plastic film as carbon source

The plastic degradability of repla1 was further demonstrated by observing the number of viable cells in the strain grown using a plastic film as the sole carbon and energy source and a microbial biofilm formed on the surface of the plastic film.

The plastic film (low-density PE and high-density PE) was added to a minimal medium, and repla1 was inoculated and incubated by shaking culture under aerobic conditions (28 °C, 130 rpm) for approximately one month. The number of viable cells in the strain was expressed as colony-forming units (CFUs).

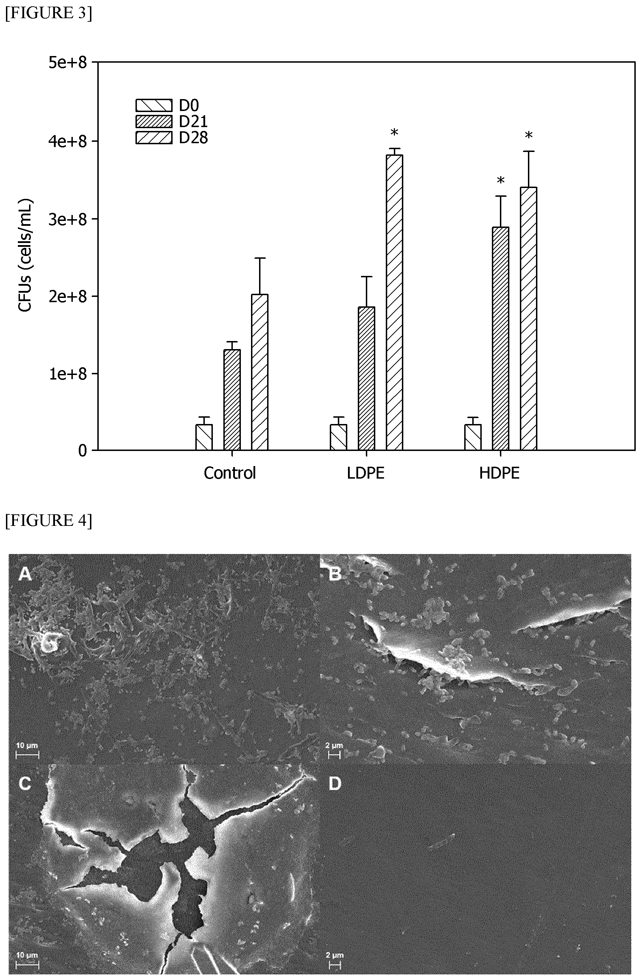

As a result of the incubation, it was able to be confirmed that the number of viable cells of repla1 increased significantly after 21 days of incubation and increased by 1.4 to 2.2 times (*p* <0.05) compared to the control on day 28 after incubation (FIG. 3).

In addition, the microbial biofilm formed on the plastic film surface and corrosion due to biodegradation were observed using a scanning electron microscope. As a result, it was able to be observed that the microbial biofilm of repla1 was formed on the plastic surface after 28 days of incubation (FIGS. 4A and 4B), and it was able to be confirmed that the plastic surface was corroded by biodegradation (FIG. 4C). In contrast, no microbial biofilm or plastic surface corrosion was observed in the control (without repla1) (FIG. 4D).

### [Reference Documents]

1. Stephen M. Jones, Edward I. Solomon. Electron Transfer and Reaction Mechanism of Laccases. Cell Mol Life Sci. 2015 Mar; 72(5): 869-883.
2. Leticia Arregui et al. Laccases: structure, function, and potential application in water bioremediation. Microb Cell Fact. 2019 Nov 14;18(1):200.
3. Zahra Montazer et al. Challenges with Verifying Microbial Degradation of Polyethylene. Polymers (Basel). 2020 Jan; 12(1): 123.
4. Zahra Montazer et al. Microbial degradation of low-density polyethylene and synthesis of polyhydroxyalkanoate polymers. Can J Microbiol. 2019 Mar;65(3):224-234.
5. Hector M. Alvarez. Relationship between β-oxidation pathway and the hydrocarbon-degrading profile in actinomycetes bacteria. International Biodeterioration & Biodegradation Volume 52, Issue 1, July 2003, Pages 35-42.
6. Masaji Watanabe et al. Computational method for analysis of polyethylene biodegradation. Journal of Computational and Applied Mathematics Volume 161, Issue 1, 1 December 2003, Pages 133-144.

### [Accession Number]

Name of Depository Institution: Korean Agricultural Culture Collection (KACC) at National Institute of Agricultural Science in Rural Development Administration
Accession Number : KACC81230BP
Deposition Date : 09/26/2022

## Claims

1. A *Pseudomonas aeruginosa* repla1 strain having plastic degrading activity, which is deposited under Accession No. KACC 81230BP.

2. The strain of claim 1, wherein the plastic is one or more selected from the group consisting of polyethylene terephthalate (PET), polyvinyl chloride (PVC), polystyrene (PS), polypropylene (PP), and polyethylene (PE).

3. The strain of claim 3, wherein the *Pseudomonas aeruginosa* repla1 strain expresses laccase.

4. A method of degrading a plastic, comprising incubating the *Pseudomonas aeruginosa* repla1 strain deposited under Accession No. KACC 81230BP with a plastic.

5. The method of claim 4, wherein the plastic is one or more selected from the group consisting of polyethylene terephthalate (PET), polyvinyl chloride (PVC), polystyrene (PS), polypropylene (PP), and polyethylene (PE).

6. The method of claim 4, wherein the incubation is performed at 8 to 40 °C.

7. A composition for degrading a plastic, comprising the *Pseudomonas aeruginosa* repla1 strain deposited under Accession No. KACC 81230BP, a culture of the strain, or a plastic-degrading enzyme derived from the strain.

8. The composition of claim 7, wherein the plastic is one or more selected from the group consisting of polyethylene terephthalate (PET), polyvinyl chloride (PVC), polystyrene (PS), polypropylene (PP), and polyethylene (PE).

9. The composition of claim 7, wherein the strain-derived plastic-degrading enzyme is laccase.
